(19) Europäisches Patentamt
European Patent Office
Office européen des brevets

(11) Publication number: **0 005 308**
**A1**

(12) # EUROPEAN PATENT APPLICATION

(21) Application number: 79300209.8

(22) Date of filing: 12.02.79

(51) Int. Cl.[2]: C 07 D 307/93

(30) Priority: 13.02.78 GB 568378

(43) Date of publication of application: 14.11.79
Bulletin 79/23

(84) Designated Contracting States: BE CH DE FR GB IT NL SE

(71) Applicant: ALLEN & HANBURYS LIMITED, Three Colts Lane Bethnal Green, London (GB)
(84) Designated Contracting States: BE CH DE FR GB IT NL SE

(71) Applicant: STANLEY MICHAEL ROBERTS, 13 Abbotts Close, Macclesfield Cheshire (GB)
(84) Designated Contracting States: BE CH DE FR GB IT NL SE

(71) Applicant: ROGER FRANK NEWTON, 22 Clarke's Way Bassingbourn, Royston Hertfordshire (GB)
(84) Designated Contracting States: BE CH DE FR GB IT NL SE

(72) Inventor: Roberts, Stanley Michael, 13, Abbotts Close, Macclesfield, Cheshire (GB)
Inventor: Newton, Roger Frank, 22 Clark's Way, Bassingbourn Royston, Hertfordshire (GB)

(74) Representative: Holmes, Michael John et al, Frank B. Dehn & Co. Imperial House 15-19 Kingsway, London WC2B 6UZ (GB)

(54) **Process for the interconversion of intermediates of use in the preparation of prostaglandins.**

(57) A process for the preparation of compounds of general formula

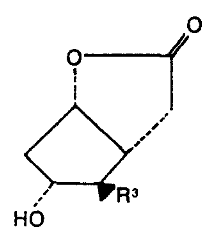

(III)

(wherein $R^3$ represents an optionally substituted $C_{1-12}$ aliphatic chain) which comprises deprotecting the group OR of a compound of formula

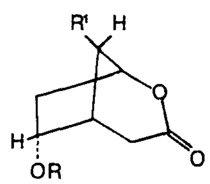

(I)

(where $R^1$ represents an optionally substituted $C_{1-12}$ aliphatic chain and R represents a hydroxyl protecting group), which process enables intermediates in the preparation of prostaglandins to be interconverted.

EP 0 005 308 A1

# Process for the interconversion of intermediates of use in the preparation of prostaglandins

This invention relates to a process of interconverting certain intermediates useful in the preparation of prostaglandins.

The prostaglandins are a class of naturally occurring cyclopentane derivatives whose importance in medicine is rapidly increasing. They are biologically active in many physiological systems and they or substances which antagonise their effects have potential application, for example, in the control of fertility, gastric secretion, blood pressure, blood coagulation and inflammation, the predominant type of activity depending on the precise chemical structure. A more detailed summary of their various activities is given in British Patent Specification No.1,396,206.

Considerable research has been carried out not only into the synthesis of natural prostaglandins but also into attempting to prepare analogues thereof having desirable

agonist or antagonist activity. In view of the complex stereochemistry of the prostaglandin molecule, such syntheses as have been developed tend to be complicated, involving a large number of steps, and means whereby the complexity of such syntheses may be reduced have considerable value. In particular, methods are required which while applicable to the manufacture of natural prostaglandins, are also applicable to the preparation of analogues and which provide flexibility in the types of prostaglandin ring structures which may be prepared.

In our Belgian Patent No. 848992 we describe $\delta$-lactones of the formula (I),

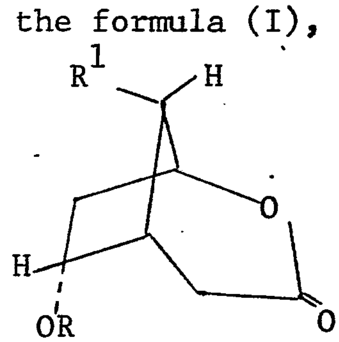

(I)

(where $R^1$ represents an optionally substituted $C_{1-12}$ aliphatic chain and R represents a hydroxyl protecting group) as intermediates useful in the synthesis, <u>inter alia</u>, of natural prostaglandins. Reduction to the corresponding lactol (hemiacetal) permits introduction of a second aliphatic chain $R^2$ to yield a product of formula (II),

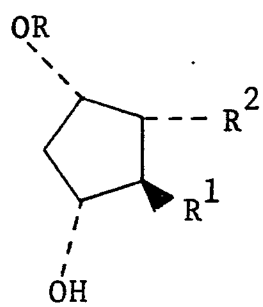

(II)

(wherein R and $R^1$ have the above meanings).

It will be appreciated that the terms A-, D-, E- and F-series as used herein refer to the substitution pattern of the cyclopentanoid ring that is characteristic of the natural prostaglandins in their defined series, without regard to the identity of the chains $R^1$ and $R^2$. The compound of formula (II) is thus a 9-protected prostaglandin of the F-series. Oxidation of the compound of formula (II) yields a corresponding compound of the D-series i.e. of the formula (IIA),

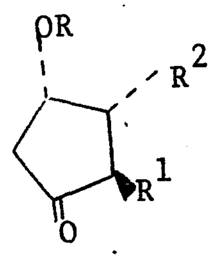

(IIA)

It is, however, tedious to convert a compound of formula (II) into a prostaglandin of the E-series, i.e. a compound of the formula (IIB),

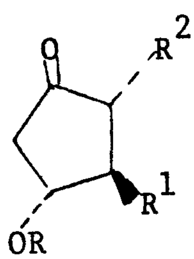

(IIB)

since this requires reversal of the protected and unprotected hydroxyl groups in the F-series compound of formula (II). Since the prostaglandins of the A- and E-series are of particular interest in medicine, it would be especially desirable if the intermediate of formula (I) could readily be converted into a compound of the E-series which in turn can afford a compound of the A-series.

We have now found that when the protected hydroxy group OR of the $\delta$-lactone of formula (I) is deprotected there is spontaneous rearrangement to the corresponding $\gamma$-lactone of formula (III),

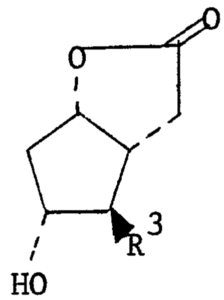

(III)

(where $R^3$ is an optionally substituted $C_{1-12}$ aliphatic chain).

Where $R^1$ carries a protected hydroxyl group, this may be deprotected in the above reaction in which case $R^3$ in the product of formula (III) will carry a hydroxyl group in place of the original protected hydroxyl group; otherwise $R^1$ and $R^3$ are identical.

The ring hydroxyl group of the compound of formula (III) and any free hydroxyl groups in $R^3$ may then be protected and the product reduced to a lactol followed by introduction of an aliphatic chain $R^2$ to yield a compound of the formuls (IV),

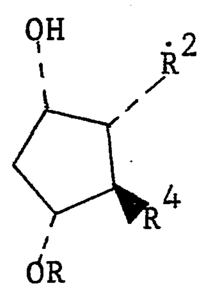

(IV)

(wherein R and $R^2$ have the above meanings and $R^4$ is the same as $R^1$ but may have different hydroxy group protection).

It will be seen that the compound of formula (IV) differs from the compound of formula (II) in that the protected and free hydroxyl groups of the ring are reversed in position. Thus oxidation of the free ring hydroxyl group in the compound of formula (IV), followed by deprotection of the other ring hydroxyl group yields a prostaglandin of the E-series and provides increased flexibility for the process of our Belgian Patent No. 848992.

The present invention relates to the removal of the protecting group R in a δ-lactone of formula (I) as defined above to form a γ-lactone of formula (III) as defined above.

As indicated, the method of the invention enables the same intermediate in the production of a prostaglandin of the D- or F-series to be used as an intermediate for the production of a prostaglandin of the A- or E-series; additionally this same intermediate permits the production of prostaglandins of the F-series having selective protection of the hydroxyl group at either the 9- or 11-position. Thus the intermediate represents a highly versatile compound.

In the formulae (I) to (IV) inclusive set out herein, a broken line - - - - connected to a ring substituent means that, with the ring substantially in the plane of the page, the substituent lies below the plane of the ring; a wedge ◀ connected to a ring substituent means that, with the ring substantially in the plane of the page, the substituent lies above the plane of the ring. Such formulae as used herein are

to be understood to depict either or both optical isomers of each of the compounds concerned as well as mixtures of said isomers, including racemates, even though the precise structure as set out relates only to one optical isomer.

The optionally substituted $C_{1-12}$ aliphatic group represented by $R^1$ may, for example, be an optionally substituted straight or branched chain alkyl, alkenyl or alkynyl group preferably having 3 or more carbon atoms. Substituents which may be present include phenyl, substituted phenyl (e.g. substituted by methyl or trifluoromethyl), $C_{3-7}$ cycloalkyl, $C_{5-7}$ cycloalkenyl, halogen (e.g. fluorine) atoms, hydroxyl, or protected or substituted hydroxyl (e.g. aliphatic or cycloaliphatic ether groups, having 1-8 carbon atoms, said aliphatic ether groups optionally containing oxygen atoms in the chain for example methoxymethyl or methoxyethoxymethyl and said cycloaliphatic ether groups optionally containing an oxygen atom in the ring as in the tetrahydropyranyloxy group; tri(hydrocarbyl)silyl ether groups such as described hereinafter in relation to the group R; aromatic ether groups such as phenoxy or substituted phenoxy e.g. substituted by halogen, methyl or trifluoromethyl; or acyloxy groups derived from $C_{1-4}$ aliphatic acids) The alkyl, alkenyl or alkynyl chain may if desired, carry an oxo or protected oxo (e.g. ketal or cyclic ketal) group, or part of the chain may form part of a cycloalkyl or cycloalkenyl ring system.

The group $R^1$ preferably a group of formula:-

$$-A^1-\underset{\underset{A^2}{\|}}{C}-A^3$$

where $A^1$ is <u>trans</u> $-CH=CB^1-$ (where $B^1$ is a hydrogen atom or methyl group); $-CH_2-CH_2-$; or $-C\equiv C-$;

$A^2$ is oxygen or a grouping of formula;

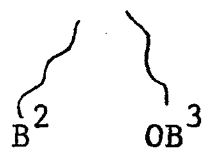

(where $B^2$ is a hydrogen atom or a methyl or ethyl, vinyl or ethynyl group, $B^3$ is hydrogen or a hydroxyl protecting group such as defined in the group R below, and a wavy line $\sim\sim$ implies either or both the R and S configurations of the carbon atom to which $B^2$ and $OB^3$ are attached);

$A^3$ is a branched or unbranched $C_{1-9}$ alkyl radical optionally substituted with an oxo group, a hydroxyl or protected hydroxyl group or one or more halogen atoms (e.g. fluorine); a branched or unbranched $C_{2-9}$ alkenyl radical; or a group $-B^4.B^5$ or $-B^4.O.B^5$

[where $B^4$ is $C_{1-5}$ alkylene, $B^5$ is $C_{5-7}$ cycloalkyl or phenyl optionally substituted by one or more halogen atoms (e.g. fluorine), $C_{1-4}$ alkyl or $C_{1-4}$ haloalkyl (e.g. trifluoromethyl)].

Particularly preferred compounds of formula (I) include those in which $R^1$ represents a group of formula

$-CH=CH-\overset{Alk^1}{\overset{|}{C}}(OR^a)-Alk^2$ or $-C\equiv C-\underset{OR^a}{\underset{|}{CH}}-Alk^3$ wherein $R^a$ has

the meaning given above for R or is a hydrogen atom,

$Alk^1$ represents a hydrogen atom or an alkyl group having up to 2 carbon atoms or a vinyl or ethynyl group, and $Alk^3$ represents a hydrogen atom or an alkyl group having up to 9 carbon atoms.

The protected hydroxyl group OR may, for example, be an acyloxy, tetrahydropyranyloxy, tri(hydrocarbyl)-silyloxy or arylmethoxy group. Where OR is an acyloxy groups this will desirably be an alkanoyloxy, aralkanoyloxy or aroyloxy group, the alkanoyloxy group preferably containing not more than 7 carbon atoms e.g. an acetoxy group and the aralkanoyloxy or aroyloxy groups preferably containing not more than 20 carbon atoms and optionally being substituted by one or more $C_{1-6}$ alkoxy groups, halogen atoms, nitro groups, $C_{1-10}$ acyloxy or $C_{2-7}$ alkoxycarbonyl groups.

Where OR is a tri(hydrocarbyl)silyloxy group this will carry three hydrocarbon substitutents which may be the same or different selected from $C_{1-6}$ alkyl, $C_{2-6}$ alkenyl, $C_{3-7}$ cycloalkyl, $C_{7-20}$ aralkyl and $C_{6-20}$ aryl groups. Such groups will include methyl, ethyl, n-propyl, isopropyl, n-butyl, isobutyl, t-butyl, allyl, phenyl and benzyl groups. Preferred hydrocarbyl groups in the hydrocarbylsilyloxy group are $C_{1-5}$ alkyl e.g. methyl and t-butyl. Thus, trimethylsilyl and t-butyldimethylsilyl ethers are particularly preferred.

Where OR is an arylmethoxy group this will desirably contain up to 20 carbon atoms, eg. a benzyloxy, diphenylmethoxy or triphenylmethoxy group.

Particularly useful groups OR include tri(hydrocarbyl)-silyloxy groups and especially t-butyldimethylsilyloxy

groups.

Deprotection of the compound of formula (I) will depend upon the nature of the group R. Where appropriate, deprotection may be carried out in the presence of either an acid or a base or by reduction. Thus, for example, an acyl group may be removed by alkaline hydrolysis. Where OR represents a tri(hydrocarbyl)silyloxy group deprotection may, for example, be effected by acid hydrolysis using, for example, dilute mineral acid or trifluoroacetic acid or by treatment with fluoride ions, employing for example, a quaternary ammonium fluoride such as tetra-n-butyl ammonium fluoride. Tetrahydropyranyl groups may be cleaved, e.g., by acid hydrolysis using, for example, a dilute mineral acid or trifluoroacetic acid. Arylmethyl groups may be removed by reduction, e.g. by using an alkali metal such as sodium dissolved in liquid ammonia or by hydrogenolysis using, for example, a noble metal catalyst such as platinum or palladium.

According to a particularly preferred embodiment of the present invention the group OR in the compound of formula (I) represents a t-butyldimethylsilyloxy group and deprotection is effected by means of tetra-n-butyl ammonium fluoride.

The subsequent rearrangement reaction occurs spontaneously to give the desired compound of formula (III) without the necessity of adding further reactants. It is particularly surprising that deprotection under conditions which would not be expected to cleave the $\delta$-lactone ring, for example acid conditions at room

temperature or using fluoride ions, nevertheless yields the γ-lactone spontaneously and, indeed with such rapidity that isolation of the unprotected δ-lactone has so far not proved possible.

The mild reaction conditions which can be used to effect the rearrangement permit relatively complex groups $R^1$ to be present, for example side chains of the type occurring in natural prostaglandins.

A number of the compounds of formula (III) are well known in the literature [e.g. Kelly and Van Rheenen (Tet. Lettr. 14 pp 1067-1070, 1976)] and, as indicated above, are valuable in the synthesis of prostaglandins and prostaglandin-type compounds.

The following non-limiting Examples serve to illustrate the present invention. The identity of all the products was additionally confirmed by conversion of each to the corresponding di-(1,1 dimethylethyl)-dimethylsilyl compound.

Example 1

Hexahydro-5-hydroxy-4-[3-hydroxy-1-octenyl]-2H-cyclopenta[b]furan-2-one[3aα, 4α (E), 5β 6aα]

8-anti-[3-(1,1-dimethylethyl)dimethylsilyloxy-1-octenyl]-6-endo-(1,1-dimethylethyl)dimethylsilyloxy-2-oxabicyclo[3,2,1]octan-3-one (0.5 g) was dissolved in tetrahydrofuran (10 ml) and tetra-n-butylammonium fluoride (1.5 g) was added thereto. The mixture obtained was heated (45°C - 50°C; 8h) and then left to stand at room temperature (25°C; 3 days). Water (20 ml) was then added thereto and the aqueous solution thus obtained was extracted with ethyl acetate (2 x 20 ml). The combined organic extracts were washed with water (2 x 20 ml) and saturated brine solution (1 x 10 ml). After drying ($MgSO_4$) evaporation of the solvent gave an oil (0.4 g) which was purified by chromatography on a column of silica gel (25 g) using methanol (1%) in ethyl acetate as the eluant. Fractions (25 ml) were collected and concentrated to yield the title compound (0.25 g) as a yellow oil from fractions 4 - 11.

TLC: EtOAc - $SiO_2$, Rf 0.23, 0.18, visualised with molybdophosphoric acid (3.5%) in ethanol.

Analysis found: C, 66.2; H, 8.6%

$C_{15}H_{24}O_4$ requires : C, 67.1; H, 9.0%

Example 2

Hexahydro-5-hydroxy-4-[3-hydroxy-1-octenyl]-2H-cyclopenta[b]furan-2-one[3aα, 4α (E), 5β, 6aα]

8-anti-[3-(1,1-dimethylethyl)dimethylsilyloxy-1-octenyl]-6-endo-(1,1-dimethylethyl)dimethylsilyloxy-2-oxabicyclo[3,2,1]octan-3-one (0.25 g), acetic acid (3 ml), water (3 ml) and tetrahydrofuran (9 ml) were heated together (60°C; 10 days). Water (15 ml) was then added thereto and the resultant mixture was extracted with ether (15 ml) and ethyl acetate (20 ml). The combined organic extracts were washed with water (15 ml) and dried ($MgSO_4$). Evaporation of the solvent gave an oil which was purified by chromatography on silica gel (25 g) using methanol (1%) in ethyl acetate as the eluant. Fractions (25 ml) were collected and concentrated to yield the <u>title compound</u> (0.1g) as a colourless oil from fractions 4-13.

<u>Example 3</u>

<u>Hexahydro-5-hydroxy-4-[3-hydroxy-1-octenyl]-2H-cyclopenta[b]furan-2-one[3aα, 4α (E), 5β, 6aα]</u>

8-<u>anti</u>-[3-(1,1-dimethylethyl)dimethylsilyloxy-1-octenyl]-6-<u>endo</u>-(1,1-dimethylethyl)dimethylsilyloxy-2-oxabicyclo[3,2,1]octan-3-one (0.8 g), hydrochloric acid (1M; 2 ml) and tetrahydrofuran (10 ml) were left to stand (3 days) at room temperature (24°C-26°C). Hydrochloric acid (5M; 0.5 ml) was then added thereto and the resultant mixture was left to stand (24 h). The mixture thus obtained was diluted with water (10 ml) and then extracted with ethyl acetate (2 x 10 ml). The combined organic extracts were washed with water and dried ($Na_2SO_4$). Evaporation of the solvent gave an oil (0.7 g) which was purified by chromatography on silica gel (50 g) using an ethyl acetate/light petroleum spirit (b.p. 60°C - 80°C) mixture (1:1) containing methanol (1%) as the eluant. The title compound (0.08 g) was obtained after concentration of the eluant as a colourless oil.

Example 4

Hexahydro-5-hydroxy-4-[3-hydroxy-1-octenyl]-2H-cyclopenta[b]furan-2-one[3aα, 4α (E), 5β, 6aα]

Crude 8-anti- [3-(1,1-dimethylethyl)dimethylsilyloxy-1-octenyl]-6-endo-(1,1-dimethylethyl)-dimethylsilyloxy-2-oxabicyclo[3,2,1]octan-3-one (2.5 g), water (5 ml), tetrahydrofuran (25 ml) and trifluoroacetic acid (3.4 ml) were left to stand (65 h) at room temperature (23°C). Water (50 ml) was then added thereto and the aqueous solution thus obtained was extracted with ether (75 ml). The ethereal extract was washed with water (3 x 75 ml) and dried ($MgSO_4$). Evaporation of the solvent gave an oil (3.2 g) which was purified on silica gel (150 g) using an ethyl acetate/light petroleum spirit (b.p. 60°C - 80°C) mixture (1:1) containing methanol (1%) as the eluant. Fractions (100 ml) were collected and concentrated to yield the title compound (0.2 g) as a coloured oil from fractions 19 - 34.

CLAIMS

1. A process for the preparation of compounds of general formula

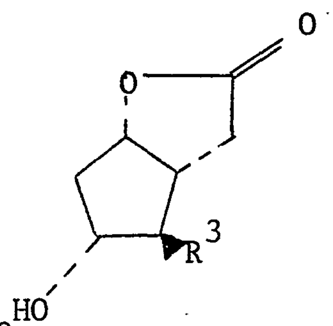

(III)

(wherein $R^3$ represents an optionally substituted $C_{1-12}$ aliphatic chain) which comprises deprotecting the group OR of a compound of formula

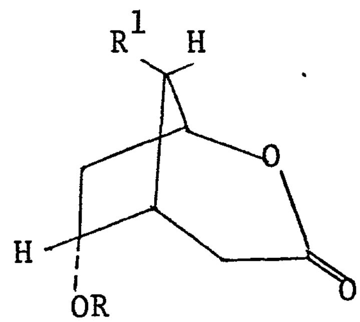

(I)

(wherein $R^1$ represents an optionally substituted $C_{1-12}$ aliphatic chain and R represents a hydroxyl protecting group).

2. A process as claimed in claim 1 wherein $R^1$ represents an optionally substituted straight or branched chain alkyl, alkenyl or alkynyl group.

3. A process as claimed in claim 2 wherein $R^1$ contains 3 or more carbon atoms.

4. A process as claimed in claim 2 or claim 3 wherein said alkyl, alkenyl or alkynyl group is substituted by a phenyl, substituted phenyl, $C_{3-7}$ cycloalkyl, $C_{5-7}$ cycloalkenyl, hydroxyl, protected or substituted hydroxyl group and/or one or more halogen atoms and/or

carries an oxo or protected oxo group or part of the group $R^1$ forms part of a cycloalkyl or cycloalkenyl ring system.

5. A process as claimed in claim 2 wherein $R^1$ represents a group of formula $-A^1-\underset{\overset{\|}{A}}{C}-A^3$ in which $A^1$ represents trans $-CH{=}CB^1-$ (where $B^1$ represents a hydrogen atom or a methyl group); $-CH_2-CH_2-$; or $-C{\equiv}C-$ :

$A^2$ represents an oxygen atom or a grouping of formula

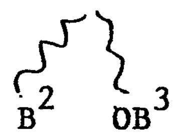

(where $B^2$ represents a hydrogen atom or a methyl, ethyl, vinyl or ethynyl group and $B^3$ represents a hydrogen atom or a hydroxyl protecting group, the wavy line ~~~ implying either or both the R and S configurations of the carbon atom to which $B^2$ and $OB^3$ are attached);

and $A^3$ represents a branched or unbranched $C_{1-9}$ alkyl radical optionally substituted with an oxo, hydroxyl or protected hydroxyl group or one or more halogen atoms; a branched or unbranched $C_{2-9}$ alkenyl radical; or a group $-B^4.B^5$ or $-B^4.O.B^5$ (where $B^4$ represents a $C_{1-5}$ alkylene group and $B^5$ represents a $C_{5-7}$ cycloalkyl or a phenyl group optionally substituted by one or more atoms or groups selected from halogen atoms, $C_{1-4}$ alkyl groups and $C_{1-4}$ haloalkyl groups).

6. A process as claimed in claim 2 wherein $R^1$ represents a group of formula

$$-CH{=}CH-\overset{\overset{Alk^1}{|}}{C}(OR^a)-Alk^2 \quad \text{or} \quad -C{\equiv}C-\underset{\underset{OR^a}{|}}{CH}-Alk^3$$

(in which $R^a$ represents a hydrogen atom or a hydroxyl protecting group, $Alk^1$ represents a hydrogen atom, an alkyl group having up to 2 carbon atoms, or a vinyl or ethynyl group, $Alk^2$ represents an alkyl group having up to 9 carbon atoms and $Alk^3$ represents a hydrogen atom or an alkyl group having up to 9 carbon atoms).

7. A process as claimed in any preceding claim wherein R represents an acyl, tetrahydropyranyl, tri(hydrocarbyl)silyl or arylmethyl group.

8. A process as claimed in claim 7 wherein R represents a t-butyldimethylsilyl group.

9. A process as claimed in claim 7 wherein R represents an acyl group and deprotection is effected by means of alkaline hydrolysis.

10. A process as claimed in claim 7 wherein R represents a tri(hydrocarbyl)silyl group and deprotection is effected by means of acid hydrolysis or by treatment with fluoride ions.

11. A process as claimed in claim 10 wherein R represents a t-butyldimethylsilyl group and deprotection is effected by treatment with tetra-n-butyl ammonium fluoride.

12. A process as claimed in claim 7 wherein R represents a tetrahydropyranyl group and deprotection is effected by means of acid hydrolysis.

13. A process as claimed in claim 7 wherein R represents an arylmethyl group and deprotection is effected by means of reduction or hydrogenolysis.

0005308 Application number

European Patent Office

# EUROPEAN SEARCH REPORT

EP 79 30 0209

## DOCUMENTS CONSIDERED TO BE RELEVANT

| Category | Citation of document with indication, where appropriate, of relevant passages | Relevant to claim |
|---|---|---|
| | DE - A - 2 704 029 (SANDOZ)<br>* Page 1, claim 1; page 8, lines 1-3 *<br>-- | 1,2,7 |
| | DE - A - 2 423 485 (ICI)<br>* Pages 35,36; claims 36,39 *<br>-- | 1-7,9 |
| X | TETRAHEDRON LETTERS, no. 49-50; December 1974, Oxford<br>J. VAN HOOLAND et al., "Cyclopentanones. XI. A novel preparation of a prostanoid synthon starting from a 3-alkyl-1,2,4-cyclopentanetrione", pages 4343-4346.<br>* Whole paper *<br>-- | 1-3,9 |
| X | BULLETIN DES SOCIETES CHIMIQUES BELGES, vol. 84, no. 8-9, August 1975, Brussels<br>W. VAN BRUSSEL et al. "Cyclopentanones. XIV. The synthesis of a prostanoid synthon starting from 3-alkyl-1,2,4-cyclopentanetriones", pages 813-830.<br>* Pages 818-819 * | 1-3,9 |

**CLASSIFICATION OF THE APPLICATION (Int. Cl.²)**

C 07 D 307/93

**TECHNICAL FIELDS SEARCHED (Int.Cl.²)**

C 07 D 307/93

**CATEGORY OF CITED DOCUMENTS**

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 18-05-1979 | ALLARD |

EPO Form 1503.1 06.78